(19) Europäisches Patentamt
European Patent Office
Office européen des brevets

(11) **EP 3 630 936 B1**

(12) **EUROPÄISCHE PATENTSCHRIFT**

(45) Veröffentlichungstag und Bekanntmachung des Hinweises auf die Patenterteilung:
**28.04.2021 Patentblatt 2021/17**

(21) Anmeldenummer: **18728553.1**

(22) Anmeldetag: **23.05.2018**

(51) Int Cl.:
*C12M 1/00* (2006.01)      *C12M 1/26* (2006.01)
*C12M 1/34* (2006.01)

(86) Internationale Anmeldenummer:
**PCT/EP2018/063470**

(87) Internationale Veröffentlichungsnummer:
**WO 2018/215521 (29.11.2018 Gazette 2018/48)**

(54) **VORRICHTUNG UND VERFAHREN ZUR KULTIVIERUNG VON ZELLEN**

DEVICE AND METHOD FOR CULTIVATING CELLS

DISPOSITIF ET PROCEDE DE MISE EN CULTURE DE CELLULES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.05.2017 DE 102017208758**

(43) Veröffentlichungstag der Anmeldung:
**08.04.2020 Patentblatt 2020/15**

(73) Patentinhaber: **AUCTEQ Biosystems**
**68167 Mannheim (DE)**

(72) Erfinder: **Kramer, Valentin**
**68259 Mannheim (DE)**

(74) Vertreter: **Geling, Andrea**
**ZSP Patentanwälte PartG mbB**
**Hansastraße 32**
**80686 München (DE)**

(56) Entgegenhaltungen:
EP-B1- 2 550 357          WO-A1-2009/093995
WO-A1-2010/114717      WO-A2-2004/106484
DE-B3-102013 002 091    US-A1- 2007 224 676
US-A1- 2016 152 935      US-B1- 6 168 944

• BENJAMIN WRIGHT ET AL: "A Novel Seed-Train Process: Using High-Density Cell Banking, a Disposable Bioreactor, and Perfusion Technologies", BIOPROCESS INTERNATI, INFORMA LIFE SCIENCES GROUP, US, Bd. 13, Nr. 3, Suppl, 15. März 2015 (2015-03-15), Seiten 16-25, XP009186510, ISSN: 1542-6319

**Beschreibung**

Die Erfindung betrifft eine Vorrichtung zur Kultivierung von Zellen umfassend eine Mischvorrichtung und einen Reaktionsraum, der von einer Reaktorwand begrenzt wird, wobei die Reaktorwand mindestens einen Anschluss für die Zugabe von Gas- und/oder Flüssigkeit aufweist und der Reaktionsraum ein inneres Volumen aufweist. Weiterhin betrifft die Erfindung ein Verfahren zur Kultivierung von Zellen in der Vorrichtung.

Bioreaktoren zur Kultivierung von Zellen umfassen als Reaktionsraum häufig Gefäße aus Glas, Edelstahl oder Kunststoff, die unterschiedliche, aber feste Größen aufweisen. Gefäße aus Glas oder Edelstahl werden üblicherweise als Mehrwegreaktoren eingesetzt und nach Gebrauch gereinigt, sterilisiert und erneut verwendet. Insbesondere Reaktionsgefäße aus Kunststoff können für den einmaligen Gebrauch bestimmt sein, der auch als Single-Use bezeichnet wird.

Weiterhin sind Bioreaktoren aus Kunststoff bekannt, die die Form einer Tasche aufweisen und im Gebrauch üblicherweise auf Schüttlern angeordnet sind. Sie werden auch als Wave-Bioreaktoren bezeichnet.

Übliche Reaktionsgefäße, insbesondere zur Kultivierung von Zellen, stellen ein vorgegebenes, festes Reaktionsvolumen zur Verfügung, das durch eine unflexible Größe der Gefäße und in der Regel auch durch eine unflexible Form der Gefäße bedingt ist.

Um einen Bioreaktor mit einem Reaktionsvolumen von mehreren Litern und insbesondere im industriellen Maßstab mit einem Reaktionsvolumen von mehr als 1 m$^3$ mit Zellen animpfen zu können muss zunächst, ausgehend von einer kleinen Menge gelagerter Zellen einer Startkultur im mL-Maßstab, eine für das große Reaktionsvolumen ausreichende Menge an Zellen hergestellt werden. Dazu werden im Rahmen einer Anzuchtschiene, die auch als seed train bezeichnet wird, schrittweise Zellen von einem kleinen Gefäß in ein nächstgrößeres Gefäß übertragen, in dem das Zellwachstum bis zum nächsten Übertrag in ein nächstgrößeres Gefäß fortgesetzt wird.

In dieser Serie von Vorkulturen befinden sich die Zellen optimalerweise insgesamt in einer exponentiellen Wachstumsphase. Das Übertragen der Zellen in größere Gefäße wird auch als Transferieren, Passagieren oder Überführen bezeichnet. Der Übertrag der Zellen in größere Volumina ist notwendig, da viele Zellen eine bestimmte Zelldichte benötigen um in die exponentielle Wachstumsphase, die auch als logarithmische Wachstumsphase oder log-Phase bezeichnet wird, einzutreten. Die Zelldichte ist üblicherweise als Anzahl Zellen pro Volumen flüssiger Zellkultur definiert. Bei Erreichen einer maximalen Zelldichte nimmt häufig das Zellwachstum, worunter die Zunahme der Anzahl der Zellen pro Zeiteinheit verstanden wird, ab. Eine Verdünnung beziehungsweise eine Zugabe weiteren Mediums ist aufgrund des festen Volumens der Gefäße nur begrenzt möglich.

Das Zellwachstum in der Anzuchtschiene wird auch als Expansion der Zellen beschrieben. Ziel der Expansion in der Anzuchtschiene ist die Bereitstellung eines Inokulums, das auch als Inokulat bezeichnet wird, das zur Inokulation, die auch als Animpfen oder Beimpfung bezeichnet wird, eines Bioreaktors im industriellen Maßstab dienen kann. Unter einer Zellexpansion, die auch als Zelllinienexpansion beschrieben wird, wird also die schrittweise Vergrößerung des Kulturvolumens und die Vermehrung der Zellen, üblicherweise über mehrere Prozessschritte hinweg, verstanden.

Häufig liegen die Zellen in einer Zellsuspension vor, wobei die Zellen und ihre extrazellulären Stoffwechselprodukte in einem üblicherweise wässrigen Nährmedium suspendiert sind.

M. Howaldt et al., in "Kultivierung von Säugetierzellen", Bioprozesstechnik, 3. Auflage, Spektrum Akademischer Verlag, 2011, Seiten 410 bis 413 beschreiben den großtechnischen Einsatz von Säugerzelltechnik, wobei das Upstream Processing aus den Kernbereichen Zellbank, Inoculum und Fermentation besteht. Zur Fermentation wird ein Aliquot aus der Zellbank aufgetaut und in einem Schüttelkolben oder in einem Spinner kultiviert. Dann werden die Zellen in nächstgrößere Kultivierungsgefäße wie Spinner, wave- oder Edelstahlbioreaktoren, transferiert, die als Vorstufen der Produktion der Zellvermehrung dienen. Mit den Anzuchtfermentern sollen möglichst schnell hohe Zellzahlen erzeugt werden, damit der Produktionsfermenter nach möglichst kurzer Zeit mit einer hohen Einsaatdichte inokuliert werden kann.

DE 10 2013 002091 offenbart eine Vorrichtung zur Kultivierung von Zellen, einen Bioreaktorbehälter mit flexibler Wandung, beispielsweise aus einer oder mehreren Polymerfolien (z.B.: PE, PP). Die EP 2 543 719 A1 beschreibt einen Mäander-Bioreaktor und ein Verfahren zur dynamischen Expansion, Differenzierung und Ernte von hämatopoetischen Zellen. Der Bioreaktor besteht aus einem Bioreaktorgefäß, dessen Bodenfläche mit versetzt angeordneten Trennwänden versehen ist, die eine mäanderartige Führung von Medien mit einer laminaren Strömung bewirken. Mehrere Mäander-Bioreaktoren können modulartig über Absperrventile in Reihe geschaltet werden. Zugeführte Zellen bilden eine die Bodenfläche nahezu bedeckende Zellschicht.

T. H. Rodriguez et al. beschreiben in "Considerations for Cell Passaging in Cell Culture Seed Trains", BMC Proceedings, 2015, 9 (Suppl 9): P43, ein softwarebasiertes Werkzeug zur Optimierung von Seed Trains, wobei ein optimaler Zeitpunkt berechnet wird, zu dem die Zellkultur von einem Maßstab in den nächstgrößeren überführt wird.

B. Wright et al. in "A Novel Seed-Train Process", BioProcess International 13(3)s, März 2015, Seiten 16 bis 25 stellen eine verbesserte Anzuchtschiene einer konventionellen Anzuchtschiene gegenüber, wobei die benötigte Zeit und die Komplexität der verbessersten Anzuchtschiene durch Verwendung einer Hochzelldichtekultur reduziert werden.

In den Richtlinien zur Handhabung von Kulturbeuteln des Herstellers Miltenyi Biotec GmbH, Handling guidelines MACS® GMP Cell Culture Bags, September 2013, sind Kulturbeutel zur Zellexpansion beschrieben, die in drei Kammern unterteilt sind, welche durch Öffnen von Schweißnähten zu einem Volumen von 100 mL miteinander verbunden werden können.

Das schrittweise Überführen der Zellen in nächstgrößere Gefäße bei der Expansion der Zellen stellt einen zeitintensiven und aufwendigen Arbeitsschritt dar. Bei der Überführung befinden sich die Zellen darüber hinaus vorübergehend außerhalb der Kulturgefäße, so dass ein erhöhtes Risiko der unerwünschten Kontamination oder Verunreinigung der Zellkultur besteht.

Aufgabe der vorliegenden Erfindung ist es eine Vorrichtung und ein Verfahren bereitzustellen, mit denen das Überführen der Zellen in mehrere Gefäße verschiedener Größe bei der Zellexpansion vermieden wird.

Gelöst wird die Aufgabe durch eine Vorrichtung zur Kultivierung von Zellen gemäß Anspruch 1 umfassend eine Mischvorrichtung und einen Reaktionsraum, der von einer Reaktorwand begrenzt wird, wobei die Reaktorwand mindestens einen Anschluss für die Zugabe von Gas und/oder Flüssigkeit aufweist und der Reaktionsraum ein inneres Volumen aufweist, wobei der Reaktionsraum so ausgestaltet ist, dass das innere Volumen um mindestens 500%, bevorzugt um mindestens 700%, mehr bevorzugt um mindestens 1000%, weiter bevorzugt um mindestens 1500% und insbesondere bevorzugt um mindestens 2000% vergrößerbar ist, bezogen auf ein minimales inneres Anfangsvolumen und wobei die Reaktorwand ein dehnbares Material aufweist, wobei das dehnbare Material eine Dehnbarkeit von mindestens 200% aufweist.

Die Aufgabe wird außerdem gelöst durch ein Verfahren zur Kultivierung von Zellen in der erfindungsgemäßen Vorrichtung gemäß Anspruch 1, umfassend folgende Schritte:

a. gegebenenfalls Sterilisieren der Reaktorwand bei einer Temperatur von 80°C bis 150°C oder durch Gammastrahlung,

b. Befüllen der Vorrichtung, wobei Zellen und gegebenenfalls ein erstes flüssiges Medium in den Reaktionsraum gegeben werden,

c. Dosieren eines zweiten flüssigen Mediums und/oder eines Gases in den Reaktionsraum, wobei bevorzugt das zweite flüssige Medium eine Zusammensetzung aufweist, die der des ersten flüssigen Mediums entspricht, und Vergrößern des inneren Volumens des Reaktionsraums um mindestens 500%, bevorzugt um mindestens 700% und mehr bevorzugt um mindestens 1000% bezogen auf das minimale innere Anfangsvolumen und wobei die Reaktorwand (16) ein dehnbares Material aufweist, wobei das dehnbare Material eine Dehnbarkeit von mindestens 200% aufweist.

Durch das vergrößerbare innere Volumen des Reaktionsraumes um mindestens 500% können größere Mengen an flüssigem Medium im selben Gefäß zugegeben werden, so dass trotz steigender Anzahl an Zellen in dem Reaktionsraum die Zelldichte bezogen auf ein Volumen an flüssigem Medium eine maximale Zelldichte nicht übersteigt und eine Überführung der Zellkultur in ein größeres Gefäß vermieden wird.

Ein erfindungsgemäß variables inneres Volumen des Reaktionsraums ist auch deshalb vorteilhaft, da neben einem vergrößerten Endvolumen auch das minimale innere Anfangsvolumen einstellbar ist. Dies ermöglicht im Vergleich zur Verwendung von großen Gefäßen zu Beginn, wenn nur eine geringe Menge an Zellkultur, die Zellen und Medium umfasst, vorliegt, ein kleineres Verhältnis von Oberfläche zum Volumen der Zellkultur. Insbesondere ist auch zu Beginn eine Phasengrenzfläche zwischen der Zellkultur, also der flüssigen Phase, und einer ebenfalls im Gefäß vorliegenden Gasphase relativ klein.

Liegt hingegen nur eine kleine Menge an Zellkultur in einem vergleichsweise großen Gefäß vor, so ist das Verhältnis von Oberfläche zum Volumen der Zellkultur groß und es kommt zu Verdunstung des Mediums bis hin zu einer Austrocknung der Zellen. Dies ist insbesondere der Fall, wenn die Kultivierung der Zellen bei einer Temperatur oberhalb der Raumtemperatur durchgeführt wird.

Zu Beginn der Zellexpansion ist das vorliegende Volumen an Zellkultur auch deshalb üblicherweise klein, da auch das Unterschreiten einer minimalen Zelldichte in der Zellkultur zu einem verminderten Zellwachstum führen kann.

Das innere Volumen des erfindungsgemäßen Reaktionsraums, wobei bereits das Zellwachstum als Reaktion aufgefasst wird, wächst im Wesentlichen entsprechend der Anzahl der im Reaktionsraum vorliegenden Zellen, bis ein gewünschtes Volumen an Zellkultur mit gewünschter Zelldichte erreicht ist. Der Reaktionsraum wird während der Expansion der Zellkultur ausgedehnt und verändert gegebenenfalls seine Form.

Durch Vermeidung der Überführung der Zellen von einem kleineren Gefäß in ein größeres Gefäß wird das Risiko einer unerwünschten Kontamination verringert. Ebenso wird Arbeitsaufwand eingespart sowie Aufwand für Material und Reinigung weiterer Gefäße.

Bevorzugt stellt das innere Volumen ein kontinuierliches Volumen dar. Ein kontinuierliches Volumen bedeutet im Rahmen der Erfindung, dass keine Unterteilung in mehrere Abschnitte vorliegt, die beispielsweise lediglich durch Rohrverbindungen oder Schlauchverbindung miteinander verbunden sind.

Als minimales inneres Anfangsvolumen wird im Rahmen der Erfindung ein kleinstes einstellbares inneres Volumen des Reaktionsraums bezeichnet. Bevorzugt liegt das minimale innere Anfangsvolumen in einem Bereich von 2 mL bis 1 L, mehr bevorzugt in einem Bereich von 5 mL bis 500 mL und insbesondere bevorzugt in einem Bereich von 5 mL bis 100 mL. Das minimale innere Anfangsvolumen ist auf ein maximales inneres Volumen des Reaktionsraums vergrößerbar und das maximale innere Volumen liegt bevorzugt in einem Bereich von 10 L bis 100 L, insbesondere bevorzugt in einem Bereich von 15 L bis 50 L.

Bevorzugt ist das innere Volumen kontinuierlich vergrößerbar. Kontinuierlich vergrößerbar bedeutet im Rahmen der Erfindung, dass das innere Volumen gleichmäßig über die Zeit zunimmt und/oder dass jedes Maß an Zunahme des inneren Volumens bis zu dem maximalen inneren Volumen stufenlos einstellbar ist.

Vorteilhaft weist die Reaktorwand ein Wandmaterial auf, das biokompatibel und nicht toxisch für Zellkulturen ist. Bevorzugt ist das Wandmaterial undurchlässig für Flüssigkeiten und stabil gegenüber einer Sterilisierung durch Hitze, Strahlung und/oder Chemikalien.

Das Wandmaterial ist bevorzugt temperaturstabil in einem Bereich von 10°C bis 150°C, mehr bevorzugt in einem Bereich von 25°C bis 130°C. Darüber hinaus ist das Wandmaterial bevorzugt stabil in Anwesenheit von Flüssigkeiten mit einem pH-Wert in einem Bereich von pH 5 bis pH 8, mehr bevorzugt in einem Bereich von pH 6 bis pH 7. Auch zeigt das Wandmaterial bevorzugt ein geringes Quellverhalten in Wasser und ist chemisch und physikalisch beständig gegenüber Wasser und wässrigen Lösungen.

Das Wandmaterial hat weiterhin bevorzugt eine Dichte in einem Bereich von 0,7 bis 1,6 $g/cm^3$, mehr bevorzugt in einem Bereich von 0,9 bis 1,6 $g/cm^3$ und insbesondere bevorzugt in einem Bereich von 0,9 bis 1,1 $g/cm^3$.

Als Wandmaterial weist die Reaktorwand ein dehnbares Material auf, wobei das dehnbare Material eine Dehnbarkeit von mindestens 200% aufweist. Erfindungsgemäß wird das dehnbare Material in Schritt c) gedehnt.

Eine Dehnung ε wird üblicherweise als relative Längenveränderung eines Körpers unter Belastung verstanden, insbesondere als Verhältnis einer Längenänderung $\Delta l$ zu einer ursprünglichen Länge $l_0$:

$$\varepsilon = \frac{\Delta l}{l_0}$$

Das dehnbare Material ist bevorzugt in mindestens zwei Raumrichtungen dehnbar, wobei die angegebene Dehnbarkeit auf mindestens eine der zwei Raumrichtungen bezogen ist. Die Dehnbarkeit beschreibt die Eigenschaft des dehnbaren Materials unter Krafteinwirkung seine Form zu verändern und gibt an, wie weit das Material gedehnt werden kann, ohne dass es bricht oder reißt. Üblicherweise wird die Dehnbarkeit, die auch als Reißdehnung bezeichnet wird, mit einem Zugversuch gemäß DIN 53504:2017-03 ermittelt.

Das Hookesche Gesetz beschreibt die Abhängigkeit der Dehnung ε von einer Spannung σ, wobei E den Elastizitätsmodul, der auch als E-Modul bezeichnet wird, darstellt:

$$\sigma = E \cdot \varepsilon$$

Bevorzugt weist das dehnbare Material einen E-Modul, der entsprechend DIN 53504:2017-03 bestimmt werden kann, in einem Bereich von 0,1 bis 0,5 GPa, mehr bevorzugt von 0,02 bis 0,1 GPa und besonders bevorzugt von 0,03 bis 0,07 GPa auf. Weiterhin beträgt die Shore Härte A des dehnbaren Materials, die gemäß DIN ISO 7619-1:2012-02 bestimmt werden kann, bevorzugt zwischen 20 und 100, mehr bevorzugt zwischen 30 und 80.

Das dehnbare Material weist bevorzugt eine hohe Elastizität auf. Als elastisch wird ein Material dann bezeichnet, wenn es bei Entlastung in den unverformten Ausgangszustand zurückkehrt, der vor der Belastung vorhanden war.

Das dehnbare Material weist weiterhin bevorzugt eine Reißfestigkeit in einem Bereich von 3,5 bis 41,4 MPa, mehr bevorzugt von 5,5 bis 35,9 MPa auf. Mehr bevorzugt weist das dehnbare Material eine Dehnbarkeit von mindestens 300% weiter bevorzugt von mindestens 330% und insbesondere bevorzugt von mindestens 500% auf. Üblicherweise weist das dehnbare Material eine Dehnbarkeit bis zu 5000% auf, bevorzugt bis zu 2000% und mehr bevorzugt bis zu 1500% auf. Ein Messmethode zur Bestimmung von Reißfestigkeit und Dehnbarkeit ist in der DIN 53504:2017-03 beschrieben.

Das dehnbare Material umfasst bevorzugt mindestens ein Elastomer, insbesondere ein thermoplastisches Elastomer. Weiterhin enthält das dehnbare Material bevorzugt ein Polymer ausgewählt aus der Gruppe bestehend aus synthetischem Kautschuk wie StyrolButadien-Kautschuk, Chloropren-Kautschuk, Polybutadien-Kautschuk, Ethylen-Propylen-Dien-Kautschuk (EPDM), Silikonkautschuk, Fluor-Kautschuk, Nitril-Kautschuk, Polyurethan (PU), hydrierter Acrylnitril-Butadien-Kautschuk (HNBR), Polypropylen, Polyisobutylen und Polyisopropen (PI), Naturkautschuk wie Latex und Mischungen davon.

Besonders bevorzugt enthält das dehnbare Material Polyisopropen, Polybutylen und/oder Silikon. Das dehnbares Material enthält bevorzugt zu mehr als 50 Gew.-%, bezogen auf das dehnbare Material, besonders bevorzugt zu mehr als 80 Gew.-% und insbesondere bevorzugt zu mehr als 90 Gew.-% Polyisopropen, Polybutylen und/oder Silikon. Bevorzugt enthält das dehnbare Material bis zu 100 Gew.-%, bezogen auf das dehnbare Material, Polyisopropen, Polybutylen und/oder Silikon und insbesondere bevorzugt besteht das dehnbare Material aus Polyisopropen, Polybutylen und/oder Silikon.

Das dehnbare Material kann außerdem Zusätze wie Proteine, insbesondere Casein und/oder Kollagen enthalten. Bevorzugt enthält das dehnbare Material Weichmacher wie Carbonsäureester, Fette, Öle und Kampfer.

Natur- und Synthesekautschuke sind insbesondere geeignet, da diese eine hohe Elastizität aufweisen. Polyurethan besitzt den Vorteil einer hohen Schmelztemperatur.

Bevorzugt ist eine Fläche der Reaktorwand, die dem Reaktionsraum zugewandt ist, vergrößerbar. Die Fläche wird bevorzugt in Schritt c) des erfindungsgemäßen Verfahrens vergrößert. Vorteilhaft wird zur Vergrößerung der dem Reaktionsraum zugewandten Fläche das dehnbare Material gedehnt. Bevorzugt wird das dehnbare Material durch Dosierung des zweiten flüssigen Mediums und/oder des Gases in den Reaktionsraum gedehnt. Die Dosierung des zweiten flüssigen Mediums und/oder des Gases erfolgt bevorzugt mittels Förderung bei erhöhtem Druck.

In einer Ausführungsform weist die Reaktorwand vorteilhaft die Form einer Mantelfläche eines Zylinders auf, die auch als Rohr bezeichnet werden kann. Eine Seite der Mantelfläche ist bevorzugt fest mit einer Bodenfläche verschlossen. Die Mantelfläche und gegebenenfalls die Bodenfläche stellen bevorzugt den zweiten Teil der Reaktorwand dar. Der erste Teil der Reaktorwand umfasst bevorzugt eine zweite Bodenfläche, die auch als Deckel bezeichnet werden kann und die beweglich an der anderen Seite der Mantelfläche, mehr bevorzugt innerhalb der Mantelfläche, angeordnet ist.

In einer weiteren Ausführungsform ist der Reaktionsraum so gestaltet, dass eine horizontale Querschnittsfläche des Reaktionsraums von unten nach oben zunimmt, wobei die Richtung von unten nach oben der Richtung der Schwerkraft entgegengesetzt ist. Bevorzugt nimmt die horizontale Querschnittsfläche des Reaktionsraums kontinuierlich zu, wobei mehr bevorzugt eine erste horizontale Querschnittsfläche des Reaktionsraums auf einer ersten Höhe mindestens doppelt so groß wie eine zweite horizontale Querschnittsfläche des Reaktionsraums auf einer zweiten Höhe ist, die erste horizontale Querschnittsfläche oberhalb der zweiten horizontalen Querschnittsfläche liegt und insbesondere bevorzugt sich ein Abstand zwischen der ersten horizontalen Querschnittsfläche und der zweiten horizontalen Querschnittsfläche befindet, der weniger als 50% einer maximalen vertikalen Ausdehnung des Reaktionsraums entspricht. Im Verlauf des erfindungsgemäßen Verfahrens nimmt der Füllstand der flüssigen Phase in dem Reaktionsraum zu, so dass die Füllhöhe in dem Reaktionsraum, dessen horizontale Querschnittsfläche von unten nach oben zunimmt, zunimmt und das Volumen der flüssigen Phase in dem Reaktionsraum, die das zweite flüssige Medium und gegebenenfalls das erste flüssige Medium umfasst, um mindestens 500%, mehr bevorzugt um mindestens 1000% und bevorzugt um nicht mehr als 10000%, bezogen auf ein minimales flüssiges Anfangsvolumen, zunimmt. Das minimale flüssige Anfangsvolumen bezeichnet das Volumen der flüssigen Phase in dem Reaktionsraum bevor in Schritt c) das zweite flüssige Medium dosiert wird. Bevorzugt liegt das minimale flüssige Anfangsvolumen in einem Bereich von 1,5 mL bis 800 mL, mehr bevorzugt in einem Bereich von 3 mL bis 400 mL und insbesondere bevorzugt in einem Bereich von 4 mL bis 100 mL. Die Füllhöhe ist auf die flüssige Phase bezogen und bezeichnet den längsten vertikalen Abstand der der Gasphase zugewandten Flüssigkeitsoberfläche von der Reaktorwand oder einem Reaktorboden. Bevorzugt verändert sich das Verhältnis der Phasengrenzfläche zwischen der flüssigen Phase und einer gasförmigen Phase in dem Reaktionsraum zum Volumen der flüssigen Phasen bei der Dosierung des zweiten flüssigen Mediums um weniger als Faktor 10, mehr bevorzugt um weniger als Faktor 5. Der Reaktionsraum ist gegebenenfalls mit einem Deckel, der bevorzugt ein flexibles Material umfasst, verschlossen. Der mindestens eine Anschluss für die Zugabe von Gas und/oder Flüssigkeit kann auch an dem Deckel angeordnet sein.

Bevorzugt hat die Reaktorwand eine kegelförmige Form, wobei die Kegelspitze nach unten, also in Richtung der Schwerkraft, weist. Ergänzend kann die Reaktorwand zumindest teilweise als Faltenbalg ausgeführt sein, wobei die Reaktorwand zumindest teilweise ziehharmonikaartig gefaltet ist.

Die Vorrichtung und insbesondere die Reaktorwand kann für den einmaligen Gebrauch oder für den mehrmaligen Gebrauch ausgestaltet sein. Im Fall des einmaligen Gebrauchs kann der Reaktionsraum auch als Einweg-Reaktor oder Single-Use-Reaktor bezeichnet werden. In diesem Fall wird insbesondere die Reaktorwand nach einmaliger Verwendung zur Kultivierung von Zellen entsorgt. Für einen ggf. mehrmaligen Gebrauch werden mindestens die Reaktorwand und der mindestens eine Anschluss für die Zugabe von Gas und/oder Flüssigkeit, bevorzugt alle Flächen der Vorrichtung zur Kultivierung von Zellen, die mit Zellen in Kontakt kommen, nach abgeschlossener Kultivierung der Zellen gereinigt, sterilisiert und wiederholt eingesetzt.

In Schritt b) des erfindungsgemäßen Verfahrens werden die Zellen und ggf. ein erstes flüssiges Medium im Reaktionsraum vorgelegt. Alternativ können auch zunächst Zellen im Reaktionsraum vorgelegt werden, die dann bevorzugt in Schritt c) des erfindungsgemäßen Verfahrens in dem zweiten flüssigen Medium suspendiert werden oder es kann zunächst das erste flüssige Medium vorgelegt werden, dem dann die Zellen zugegeben werden.

Bevorzugt weisen das erste flüssige Medium und das zweite flüssige Medium eine gleiche Zusammensetzung auf. Eine gleiche Zusammensetzung umfasst auch den Fall, dass ein Gehalt an extrazellulären Stoffwechselprodukten der Zellen in den ersten und zweiten flüssigen Medium abweichend ist. So können die Zellen in einer Suspension im ersten flüssigen Medium vorgelegt werden, die dann durch Zugabe des zweiten flüssigen Mediums lediglich verdünnt werden, so dass sich die Zellsuspension im Wesentlichen vor und nach dem Schritt c) lediglich in der Zelldichte, nicht aber in der Zusammensetzung des Mediums unterscheidet.

In einer alternativen Ausführungsform kann die Zusammensetzung des ersten flüssigen Mediums von der Zusammensetzung des zweiten flüssigen Mediums abweichen. So kann beispielsweise die Zusammensetzung des zweiten flüssigen Mediums an die fortgeschrittene Wachstumsphase der Zellen angepasst sein.

Das zweite flüssige Medium und ggf. das erste flüssige Medium enthalten bevorzugt mindestens 50 Gew.-% Wasser, mehr bevorzugt mindestens 75 Gew.-% und insbesondere bevorzugt mindestens 90 Gew.-% Wasser. Bevorzugt enthalten das zweite flüssige Medium und ggf. das erste flüssige Medium bis zu 99 Gew.-% Wasser.

Weiterhin enthalten das zweite flüssige Medium und ggf. das erste flüssige Medium bevorzugt anorganische Salze wie $CaCl_2$, $Fe(NO_3)_3$, KCl, $MgSO_4$, NaCl, $NaH_2PO_4$ und/oder $NaHCO_3$, Aminosäuren wie L-Arginin, L-Cystin, L-Glutamin, Glycin, L-Histidin, L-Isoleucin, L-Leucin, L-Lysin, L-Methionin, L-Phenylalanin, L-Serine, L-Threonin, L-Tryptophan, L-Thyrosin und/oder L-Valin, Vitamine wie D-Calciumpantothenat, Cholinchlorid, Folsäure, i-Inositol, Niacinamid, Riboflavin und/oder Thiamin, und/oder andere Komponenten wie D-Glucose, Phenolrot und/oder Natriumpyruvat.

Besonders bevorzugt enthalten das zweite flüssige Medium und ggf. das erste flüssige Medium mindestens 50 Gew.-% Dulbecco's Modified Eagle's Medium (DMEM), mehr bevorzugt mindestens 75 Gew.-% und insbesondere bevorzugt mindestens 90 Gew.-% DMEM.

Das zweite flüssige Medium wird bevorzugt kontinuierlich zugegeben. Alternativ kann die Zugabe des zweiten flüssigen Mediums schrittweise, was auch als portionsweise bezeichnet werden kann, erfolgen. Die Zuführungsrate bei der kontinuierlichen Zuführung kann konstant sein oder an das Zellwachstum angepasst werden. Bei schrittweiser Zugabe können der Zeitpunkt und das zugegebene Volumen pro Schritt an das Zellwachstum angepasst sein oder jeweils nach einem festen Zeitintervall und mit einem festen Volumen erfolgen.

Aufgrund der Zugabe des zweiten flüssigen Mediums kann das erfindungsgemäße Verfahren auch als Fed-Batch-Verfahren bezeichnet werden.

Weiterhin kann auch zusätzlich weiteres Gas und/oder weitere Flüssigkeit in den Reaktionsraum gegeben werden. Die Reaktorwand weist bevorzugt mindestens zwei Anschlüsse für die Zugabe von Gas und/oder Flüssigkeit auf. Vorteilhaft erfolgt die Dosierung des zweiten flüssigen Mediums und/oder des Gases in Schritt c) durch die Anschlüsse, wobei besonders bevorzugt das zweite flüssige Medium und/oder das Gas jeweils separat durch einen Anschluss erfolgt. Insbesondere kann die Reaktorwand Anschlüsse für Zuluft und/oder Sauerstoff, Kohlenstoffdioxid, Stickstoff und/oder Abluft aus dem Reaktionsraum umfassen. Die Vorrichtung zur Kultivierung von Zellen umfasst bevorzugt Anschlüsse, die Sterilfilter aufweisen. Bevorzugt wird der Reaktionsraum kontinuierlich begast und kontinuierlich Gas aus dem Reaktionsraum abgeführt, so dass ein kontinuierlicher Austausch der Gasphase im Reaktionsraum stattfindet.

Weiterhin können Anschlüsse, bevorzugt ohne Sterilfilter, vorliegen, die den Reaktionsraum mit sterilen Vorratsbehältern verbinden, so dass Anschlüsse zur Zugabe von weiteren Flüssigkeiten insbesondere Nährmedium vorliegen. Auch kann ein Anschluss zur Probenentnahme vorgesehen sein.

Bevorzugt weist die Reaktorwand ein Anschlussstück auf, an dem der mindestens eine Anschluss für die Zugabe von Gas und/oder Flüssigkeit angeordnet ist.

Bevorzugt werden die Zellen, das zweite flüssige Medium und ggf. das erste flüssige Medium in dem Reaktionsraum gemischt, insbesondere mit Hilfe der Mischvorrichtung. Die Mischvorrichtung kann einen Mischer mit rotierender Welle, einen Vibrationsmischer, einen hydraulischen Mischer, einen pneumatischen Mischer, einen statischen Mischer oder einen Schüttler umfassen.

Beispielsweise ist die Mischvorrichtung in dem Reaktionsraum in Form eines Rührers, insbesondere in Form eines Magnetrührers angeordnet. Bevorzugt ist der Reaktionsraum auf einem Schüttler wie einer Schüttelplatte oder einem Orbitalschüttler angeordnet. Weiter bevorzugt ist der Reaktionsraum mit einem Befestigungsmittel auf dem Schüttler befestigt, wobei das Befestigungsmittel bevorzugt dehnbar ist. Mit Hilfe der Mischvorrichtung wird das zweite flüssige Medium und ggf. das erste flüssige Medium enthaltend die Zellen bevorzugt in eine Wellenbewegung versetzt.

Der Reaktionsraum enthält bevorzugt die gasförmige Phase und die flüssige Phase, wobei die flüssige Phase, die bevorzugt eine Suspension ist, das zweite flüssige Medium, die Zellen und ggf. das erste flüssige Medium umfasst. Bevorzugt bleibt das Volumenverhältnis der gasförmigen Phase und der flüssigen Phase im Reaktionsraum im Wesentlichen unverändert, was durch die Vergrößerung des inneren Volumens beim Dosieren des zweiten flüssigen Mediums und/oder des Gases möglich ist. Auf diese Weise bleibt auch die Größe der Phasengrenzfläche zwischen der flüssigen Phase und der gasförmigen Phase im Verhältnis zum Volumen der flüssigen Phase im Wesentlichen konstant, so dass auch Verdunstungseffekte über die Kultivierungszeit im Wesentlichen unverändert bleiben.

Bevorzugt nimmt vor dem Dosieren in Schritt c) die flüssige Phase 20 Vol.-% bis 80 Vol.-%, mehr bevorzugt 30 Vol.-% bis 70 Vol.-% und insbesondere bevorzugt 40 Vol.-% bis 60 Vol.-% des inneren Volumens ein. Weiterhin nimmt bevorzugt nach dem Dosieren in Schritt c) die flüssige Phase 10 Vol.-% bis 80 Vol.-%, mehr bevorzugt 20 Vol.-% bis 60 Vol.-% und insbesondere bevorzugt 30 Vol.-% bis 50 Vol.-% des inneren Volumens ein. Die Anwesenheit der gasförmigen Phase in dem Reaktionsraum, die bevorzugt Sauerstoff enthält, ist insbesondere bei der Kultivierung von aeroben Zellen vorteilhaft.

Bevorzugt liegen die Zellen in dem Reaktionsraum in Suspensionskultur vor. Die Zellen umfassen insbesondere nicht-adhäsive Zellen, was dahingehend zu verstehen ist, dass die Zellen im Wesentlichen nicht an der Reaktorwand oder anderen Flächen im Reaktionsraum anhaften.

Bevorzugt umfassen die Zellen eukaryotische Zellen, die in Suspensionskultur vorliegen, insbesondere Säugerzellen, Pflanzenzellen, Insektenzellen und/oder Pilzzellen, besonders bevorzugt Säugerzellen. Alternativ oder zusätzlich können die Zellen auch prokaryotische Zellen wie Bakterien umfassen.

Es ist ebenso denkbar, die Vorrichtung zur Kultivierung von adhärenten Zellen zu nutzen, so dass die im Reaktionsraum vorliegenden Zellen adhärente Zellen umfassen.

In Schritt c) des erfindungsgemäßen Verfahrens nimmt bevorzugt die Anzahl der Zellen, die im Reaktionsraum vorliegen, durch Zellwachstum zu. Dazu wird der Reaktionsraum bevorzugt auf eine Temperatur im Bereich von 15°C bis 50°C, mehr bevorzugt von 20°C bis 45°C, weiter bevorzugt von 35°C bis 40°C und insbesondere bevorzugt von 36°C bis 48°C, temperiert.

Kurze Beschreibung der Zeichnungen

Ein Ausführungsbeispiel gemäß dem Stand der Technik und erfindungsgemäße Ausführungsformen sind in den Zeichnungen dargestellt und werden in der nachfolgenden Beschreibung näher erläutert.

Es zeigen:

Figur 1    eine schematische Darstellung einer üblichen Anzuchtschiene;

Figur 2    eine erfindungsgemäße Vorrichtung zur Kultivierung von Zellen mit einer Reaktorwand umfassend ein dehnbares Material;

Figur 3    eine erfindungsgemäße Vorrichtung zur Kultivierung von Zellen mit einer kegelförmigen Reaktorwand,

Figur 4    eine Seitenansicht eines Reaktionsraums mit einer Reaktorwand umfassend ein dehnbares Material,

Figur 5    eine weitere Ansicht des Reaktionsraums mit einer Reaktorwand umfassend ein dehnbares Material,

Figur 6    eine schematische Darstellung einer Vorrichtung zur Kultivierung von Zellen mit einem Reaktionsraum mit einer Reaktorwand aus einem dehnbaren Material.

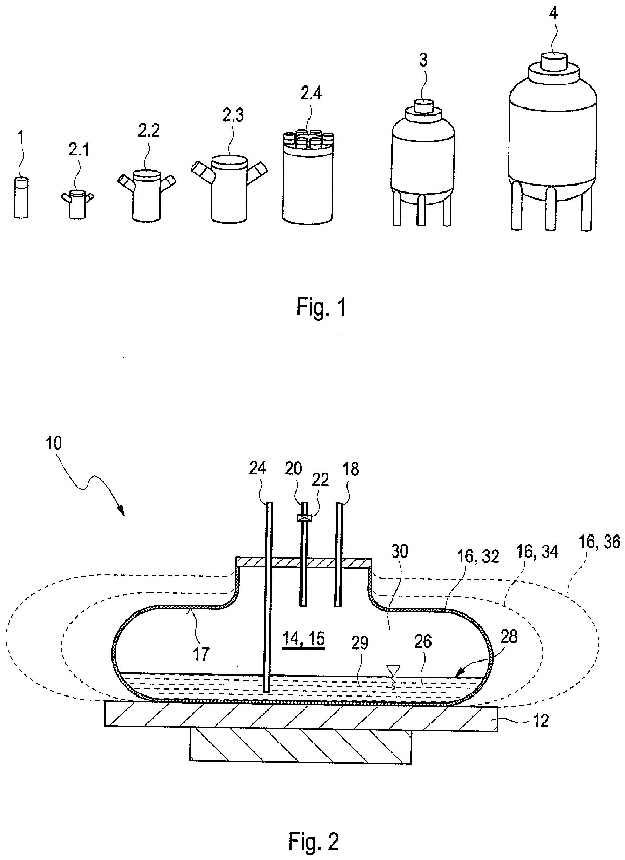

Figur 1 zeigt in schematischer Darstellung den Ablauf einer üblichen Anzuchtschiene. Aus einem Lagergefäß 1, das eine Startkultur enthält, wird eine Zellkultur in Form einer Zellsuspension, die Zellen und ein Kulturmedium umfasst, mit einem Volumen von 1 mL und einer Zelldichte von $2x10^7$ Zellen/mL in ein erstes Expansionsgefäß 2.1 gegeben. Insgesamt werden vier Expansionsgefäße 2.1, 2.2, 2.3 und 2.4 eingesetzt um ausreichend Zellen als Inokulum zur Animpfung eines Produktionsgefäßes 4 herstellen zu können. Da eine maximale volumenspezifische Zelldichte nicht überschritten werden sollte, um das Zellenwachstum nicht zu behindern, wird der Zellsuspension während der Expansion mit zunehmender Anzahl an Zellen frisches Medium zugegeben.

Ist in dem ersten Expansionsgefäß 2.1 ein maximaler Füllstand erreicht, wird die Zellsuspension vom ersten Expansionsgefäß 2.1 in das zweite Expansionsgefäß 2.2 überführt und entsprechend dem Zellwachstum weiteres frisches Medium zugegeben. Die Zellsuspension wird anschließend entsprechend in das dritte Expansionsgefäß 2.3 und dann in das vierte Expansionsgefäß 2.4 überführt.

Vom ersten Expansionsgefäß 2.1 zum vierten Expansionsgefäß 2.4 wird das Volumen der Zellsuspension von 125 mL auf 10 L erhöht. Mit der Zellsuspension, die sich im vierten Expansionsgefäß 2.4 befindet, wird ein Vorproduktionssgefäß 3 angeimpft, das ein inneres Volumen von 50 L aufweist und auch als n-1 Stufe bezeichnet wird. Im Vorproduktionssgefäß 3 nimmt die Anzahl der Zellen weiter zu, so dass der Inhalt des Vorproduktionssgefäßes 3 zur Animpfung des Produktionsgefäßes 4 dient, das ein inneres Volumen von 500 L aufweist. Bei den hier dargestellten Gefäßen handelt es sich um Satzreaktoren, die auch als Batchreaktoren bezeichnet werden.

Figur 2 zeigt eine schematische Darstellung einer erfindungsgemäßen Vorrichtung zur Kultivierung von Zellen 10 mit einer Reaktorwand 16 umfassend ein dehnbares Material. Es ist eine Vorrichtung zur Kultivierung von Zellen 10 dargestellt, die die vier Expansionsgefäße 2.1, 2.2, 2.3 und 2.4 gemäß Figur 1 ersetzen kann, da die erforderliche Volumenzunahme der Zellsuspension in demselben Gefäß möglich ist.

Die Vorrichtung zur Kultivierung von Zellen 10 umfasst eine Mischvorrichtung 12, auf der ein Reaktionsraum 14 angeordnet ist. Die Mischvorrichtung 12 ist beispielsweise ein Orbitalschüttler. Der Reaktionsraum 14 mit einem inneren Volumen 15 wird von einer Reaktorwand 16 begrenzt. Die Reaktorwand 16 umfasst einen Anschluss für die Zugabe von Flüssigkeit 18 und einen Anschluss für die Zugabe von Gas 20, wobei der Anschluss für die Zugabe von Gas 20 einen Sterilfilter 22 aufweist. Zusätzlich ist ein Schlauch 24 zur Probeentnahme vorgesehen.

Der Reaktionsraum 14 enthält sowohl eine flüssige Phase 28 als auch eine gasförmige Phase 30. Die flüssige Phase 28 enthält ein zweites flüssiges Medium 26 und ggf. auch ein erstes flüssiges Medium sowie Zellen 29.

In einem Ausgangszustand 32 weist der Reaktionsraum 14 ein minimales inneres Anfangsvolumen auf und durch Zugabe von zweitem flüssigem Medium 28 und/oder Gas durch den Anschluss zur Zugabe von Flüssigkeit 18 und/oder durch den Anschluss zur Zugabe von Gas 20 wird die Reaktorwand 16, die in dieser Ausführungsform ein dehnbares Material aufweist, gedehnt, so dass ein erster Dehnungszustand 34 erreicht wird. Durch Dehnung des dehnbaren Materials wird eine dem Reaktionsraum zugewandte Fläche 17 der Reaktorwand 16 vergrößert. Durch weitere Zugabe von zweitem flüssigem Medium 26 oder Gas wird die Reaktorwand 16 weiter gedehnt, so dass ein zweiter Dehnungszustand 36 erreicht wird.

Im Gegensatz zu den Behältern 2.1, 2.2, 2.3 und 2.4 gemäß Figur 1 nimmt in der erfindungsgemäßen Ausführungsform nach Figur 2 nicht nur das Volumen der flüssigen Phase 28, also der Zellsuspension, zu, sondern auch das innere Volumen 15 des Reaktionsraums 14, so dass ein Umfüllen der flüssigen Phase 28 in ein nächstgrößeres Gefäß entfällt bis ein maximales inneres Volumen des erfindungsgemäßen Reaktionsraums 14 erreicht wird. Entsprechend kann die Zellexpansion in nur einem Gefäß durchgeführt werden.

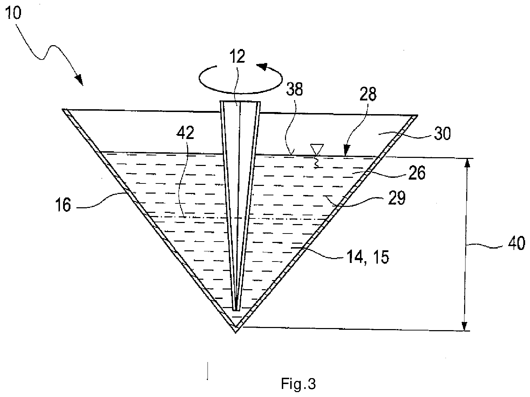

Figur 3 zeigt eine erfindungsgemäße Vorrichtung zur Kultivierung von Zellen 10 mit einer kegelförmigen Reaktorwand 16. Die Vorrichtung zur Kultivierung von Zellen 10 umfasst eine Mischvorrichtung 12 in Form eines Rührers. Der Reaktionsraum 14 mit einem inneren Volumen 15 weist eine kegelförmige Form auf.

In dem Reaktionsraum 14, dessen horizontale Querschnittsfläche 42 von unten nach oben zunimmt, befindet sich sowohl eine flüssige Phase 28 umfassend das zweite flüssige Medium 26, gegebenenfalls das erste flüssige Medium und Zellen 29, sowie eine gasförmige Phase 30. Die flüssige Phase 28 erreicht eine Füllhöhe 40. Die Kegelform ermöglicht im Vergleich zu anders geformten Reaktorwänden 16, dass auch bei kleinen Volumina der flüssigen Phase 28 ein ausreichend kleines Verhältnis der Phasengrenzfläche 38, die sich zwischen der flüssigen Phase 28 und der gasförmigen Phase 30 befindet, zum Volumen der flüssigen Phase 28 vorliegt, so dass Verdunstungseffekte nicht vorherrschend sind. Dennoch ist eine erforderliche Zunahme des Volumens der flüssigen Phase 28 in der Zellexpansion möglich.

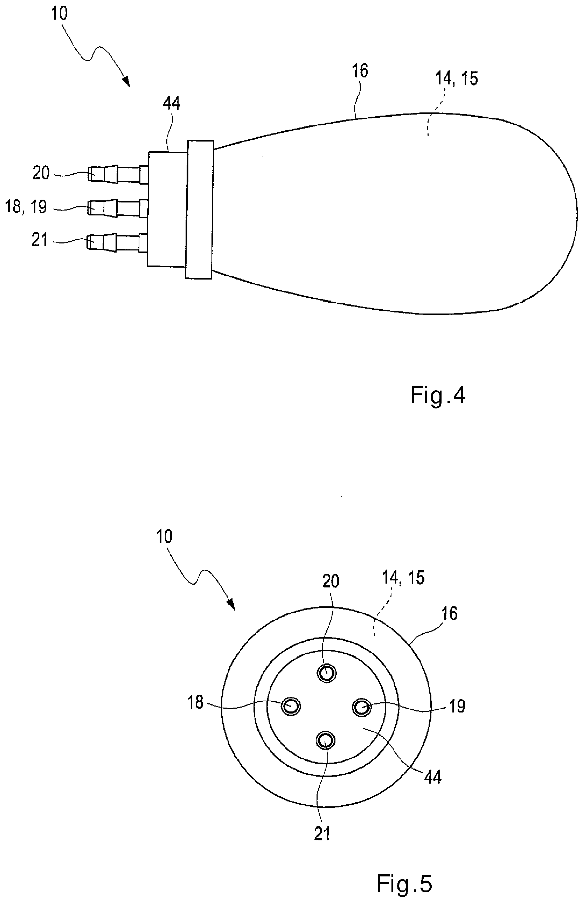

Figur 4 zeigt eine Seitenansicht eines Reaktionsraums 14 mit einer Reaktorwand 16 umfassend ein dehnbares Material. Der Reaktionsraum 14 weist ein inneres Volumen 15 auf, das durch Dehnung des dehnbaren Materials vergrößerbar ist. Die Reaktorwand 16 weist ein Anschlussstück 44 auf, an dem ein Anschluss für die Zugabe von Flüssigkeit 18, ein Anschluss zur Probenahme 19, ein Anschluss für die Zugabe von Gas 20 und ein Gasauslass 21 angeordnet sind.

Figur 5 zeigt eine weitere Ansicht des Reaktionsraums 14 mit der Reaktorwand 16 umfassend ein dehnbares Material gemäß Figur 5. Das Anschlussstück 44 weist einen Anschluss für die Zugabe von Flüssigkeit 18, einen Anschluss zur Probenahme 19, einen Anschluss für die Zugabe von Gas 20 und einen Gasauslass 21 auf.

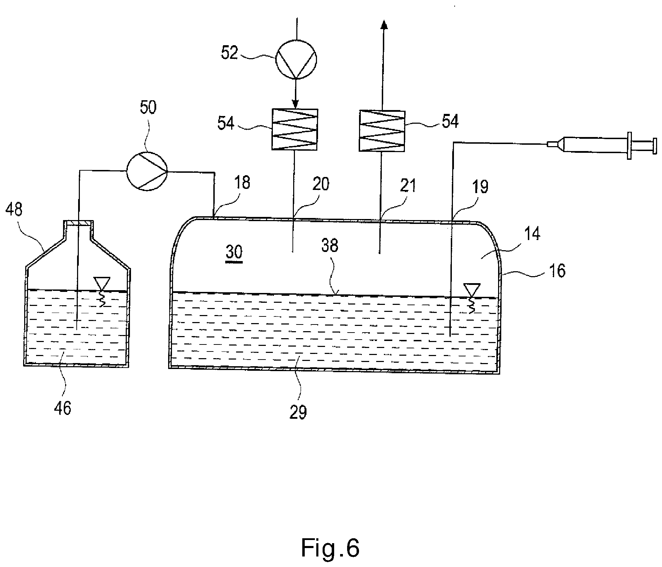

In Figur 6 ist eine Vorrichtung zur Kultivierung von Zellen mit einem Reaktionsraum aus einem dehnbaren Material schematisch dargestellt.

Der Reaktionsraum 14 aus dehnbarem Material kann dabei zum Beispiel wie in den Figuren 4 und 5 dargestellt aufgebaut sein. Alternativ und bevorzugt ist es jedoch, wenn der Reaktionsraum 14 vollständig von einer von einer Reaktorwand 16 aus dehnbarem Material umschlossen ist und der Anschluss für die Zugabe von Flüssigkeit 18, der Anschluss für die Zugabe von Gas 20, der Gasauslass 21 und der Anschluss zur Probennahme 19 direkt durch das die Reaktorwand bildende dehnbare Material geführt werden. Die Abdichtung des Anschlusses für die Zugabe von Flüssigkeit 18, des Anschlusses für die Zugabe von Gas 20, des Gasauslasses 21 und des Anschlusses zur Probennahme 19 erfolgt dabei auf dem Fachmann bekannte übliche Weise, zum Beispiel durch Einkleben.

Zur Kultivierung von Zellen wird Kulturmedium 46 aus einem Vorratsgefäß 48 mittels einer Pumpe 50, vorzugsweise einer pneumatischen Pumpe, über den Anschluss für die Zugabe von Flüssigkeit 18 in den Reaktionsraum transportiert. Die Förderleistung der Pumpe ist dabei abhängig vom Wachstum der im Reaktionsraum 14 kultivierten Zellen 29. Hierbei kann das Kulturmedium 46 entweder kontinuierlich in den Reaktionsraum 14 gefördert werden oder auch schrittweise, wobei jeweils dann Kulturmedium nachgeführt wird, wenn das im Reaktionsraum enthaltene nahezu aufgebraucht ist. Alternativ zu einer pneumatischen Pumpe kann auch jede beliebige andere Pumpe eingesetzt werden, mit der ein kontaminationsfreier Transport des Kulturmediums 46 in den Reaktionsraum 14 möglich ist.

Über den Anschluss für die Zugabe von Gas 20 wird das für die Kultivierung der Zellen notwendige Gas, üblicherweise ein Sauerstoff enthaltendes Gas wie reiner Sauerstoff oder Luft oder auch mit Sauerstoff angereicherte Luft dem Reaktionsraum zugeführt. Die Zugabe des Gases erfolgt dabei mittels einer Gaspumpe 52. Um zu verhindern, dass Verunreinigungen mit dem Gas in den Reaktionsraum eingetragen werden, wird das Gas vor der Zugabe in den Reaktionsraum durch einen Sterilfilter 54 geleitet. Als Gaspumpe 52 eignet sich zum Beispiel eine Membranpumpe. Es kann jedoch auch hier jede beliebige andere geeignete Pumpe eingesetzt werden, mit der Gas gefördert werden kann.

Überschüssiges Gas kann durch den Gasauslass 21 aus dem Reaktionsraum 14 entnommen werden. Um zu verhindern, dass mit dem Gas unerwünschte Komponenten aus dem Reaktionsraum 14 ausgetragen werden, schließt sich an den Gasauslass 21 ebenfalls ein Sterilfilter 54 an.

Der Anschluss zur Probennahme 19 ist mit einem geeigneten Probenentnahmesystem 56 verbunden. Damit auch hier keine Kontaminationen in das System eingebracht werden können und auch die entnommenen Proben nicht verunreinigt werden, ist es notwendig, dass das Probenentnahmesystem 56 steril ist. Um eine Probe aus dem Reaktionsraum 14 zu entnehmen, ist es notwendig, dass am Anschluss zur Probennahme 19 ein Druck angelegt werden kann, der unterhalb des Drucks im Reaktionsraum 14 liegt, so dass die Probe aufgund der Druckdifferenz aus dem Reaktionsraum 14 entnommen wird. Als Probenentnahmesystem eignet sich zum Beispiel eine Spritze, an deren Spritzenkolben zur Probenentnahme gezogen wird, so dass sich der Raum zur Aufnahme der Probe während der Probenentnahme vergrößert.

Beispiel 1

In einer Vorrichtung zur Kultivierung von Zellen gemäß Figur 2, die ein dehnbares Material als Wandmaterial aufwies, wurde eine flüssige Phase mit einem Volumen von 10 mL vorgelegt. Über zehn Stunden wurden kontinuierlich ein wässriges Medium und Luft zugeführt, bis die flüssige Phase in dem Reaktionsraum ein Volumen von 20 L erreichte.

Der Versuch wurde mit drei unterschiedlichen Wandmaterialien durchgeführt. Die Reaktorwand bestand jeweils im Wesentlichen aus Naturkautschuk, Polyisopren oder Polyurethan, welche jeweils eine gute Biokompatibilität aufweisen. Die Reaktorwand wies zu Beginn vor der Ausdehnung eine Wanddicke von etwa 100 $\mu$m auf.

Mit allen drei Materialien wurde eine Volumenausdehnung des inneren Volumens von mindestens 2000% erreicht. Die Reaktorwand aus Polyisopren und die Reaktorwand aus Naturkautschuk wiesen eine noch höhere Dehnbarkeit als die Reaktorwand aus Polyurethan auf.

Beispiel 2

In einer Vorrichtung zur Kultivierung von Zellen gemäß Figur 2, die eine Reaktorwand im Wesentlichen bestehend aus Polyisopren aufwies, wurden 500 mL einer wässrigen Suspension enthaltend Hefezellen (Saccharomyces cerevisiae) vorgelegt. Die Zellen wurden über einen Zeitraum von 14 Tagen kultiviert, während zusätzlich insgesamt 4,5 L wässriges Medium schrittweise zugegeben wurden. Im gesamten Kultivierungszeitraum konnte die Produktion von Metaboliten beobachtet werden.

Beispiel 3

Eine Vorrichtung zur Kultivierung von Zellen gemäß Figur 2, die eine Reaktorwand im Wesentlichen bestehend aus Polyisopren aufwies, wurde bei 120°C und 2 bar für 2 Stunden autoklaviert. 50 mL Zellkulturmedium, bestehend aus Dulbecco's Modified Eagle's Medium (DMEM), enthaltend 10 Gew.-% Fötales Kälberserum (FKS), wurden in der autoklavierten Vorrichtung bei 37°C für 7 Tage inkubiert, dann wurde eine Probe des Zellkulturmediums unter dem Mikroskop betrachtet. Bei einer 10.000fachen Vergrößerung wurden keine Zellen beobachtet. Folglich war die Vorrichtung zum sterilen Arbeiten geeignet.

<div align="center">Bezugszeichenliste</div>

| | | | | |
|---|---|---|---|---|
| 1 | Lagergefäß | | 52 | Gaspumpe |
| 2 | Expansionsgefäße | | 54 | Sterilfilter |
| 2.1 | erstes Expansionsgefäß | | 56 | Probenentnahmesystem |
| 2.2 | zweites Expansionsgefäß | | | |
| 2.3 | drittes Expansionsgefäß | | | |
| 2.4 | viertes Expansionsgefäß | | | |
| 3 | Vorproduktionsgefäß | | | |
| 4 | Produktionsgefäß | | | |
| 10 | Vorrichtung zur Kultivierung von Zellen | | | |
| 12 | Mischvorrichtung | | | |
| 14 | Reaktionsraum | | | |
| 15 | inneres Volumen | | | |
| 16 | Reaktorwand | | | |
| 16.1 | erster Teil | | | |

(fortgesetzt)

| 16.2 | zweiter Teil |
|------|--------------|
| 17 | dem Reaktionsraum zugewandte Fläche |
| 18 | Anschluss für die Zugabe von Flüssigkeit |
| 19 | Anschluss zur Probenahme |
| 20 | Anschluss für die Zugabe von Gas |
| 21 | Gasauslass |
| 22 | Sterilfilter |
| 24 | Schlauch |
| 26 | zweites flüssiges Medium |
| 28 | flüssige Phase |
| 29 | Zellen |
| 30 | gasförmige Phase |
| 32 | Ausgangszustand |
| 34 | erster Dehnungszustand |
| 36 | zweiter Dehnungszustand |
| 38 | Phasengrenzfläche |
| 42 | horizontale Querschnittsfläche |
| 44 | Anschlussstück |
| 46 | Kulturmedium |
| 48 | Vorratsgefäß |
| 50 | Pumpe |

**Patentansprüche**

1. Vorrichtung zur Kultivierung von Zellen (10) umfassend eine Mischvorrichtung (12) und einen Reaktionsraum (14), der von einer Reaktorwand (16) begrenzt wird, wobei die Reaktorwand (16) mindestens einen Anschluss (18, 20) für die Zugabe von Gas und/oder Flüssigkeit aufweist und der Reaktionsraum (14) ein inneres (15) Volumen aufweist, wobei der Reaktionsraum (14) so ausgestaltet ist, dass das innere Volumen (15) um mindestens 500% vergrößerbar ist, bezogen auf ein minimales inneres Anfangsvolumen, und
wobei die Reaktorwand (16) ein dehnbares Material aufweist, wobei das dehnbare Material eine Dehnbarkeit von mindestens 200% aufweist.

2. Vorrichtung nach Anspruch 1, **dadurch gekennzeichnet, dass** das dehnbare Material Naturkautschuk, synthetischen Kautschuk, Polyisobutylen, Silikonkautschuk oder Mischungen davon enthält.

3. Vorrichtung nach den Ansprüchen 1 oder 2, **dadurch gekennzeichnet, dass** das minimale innere Anfangsvolumen in einem Bereich von 2 mL bis 1 L liegt und auf ein maximales inneres Volumen von 10 L bis 100 L vergrößerbar ist.

4. Vorrichtung nach einem der Ansprüche 1 bis 3, **dadurch gekennzeichnet, dass** der mindestens eine Anschluss (18, 20) für die Zugabe von Gas und/oder Flüssigkeit durch das dehnbare Material der Reaktorwand (16) geführt ist.

5. Verfahren zur Kultivierung von Zellen in einer Vorrichtung gemäß einem der Ansprüche 1 bis 4, umfassend folgende Schritte:

    a. gegebenenfalls Sterilisieren der Reaktorwand (16) bei einer Temperatur von 80°C bis 150°C oder durch Gammastrahlung,
    b. Befüllen der Vorrichtung, wobei Zellen (29) und gegebenenfalls ein erstes flüssiges Medium in den Reaktionsraum (14) gegeben werden,
    c. Dosieren eines zweiten flüssigen Mediums (26) und/oder eines Gases in den Reaktionsraum (14), wobei bevorzugt das zweite flüssige Medium eine Zusammensetzung aufweist, die der des ersten flüssigen Mediums (26) entspricht,
    und
    Vergrößern des inneren Volumens (15) des Reaktionsraums (14) um mindestens 500% bezogen auf das minimale innere Anfangsvolumen,
wobei die Reaktorwand (16) ein dehnbares Material aufweist und das dehnbare Material in Schritt c. gedehnt wird.

**6.** Verfahren nach Anspruch 5, **dadurch gekennzeichnet, dass** das zweite flüssige Medium (26) kontinuierlich und/oder schrittweise zugeben wird.

**7.** Verfahren nach den Ansprüchen 5 oder 6, **dadurch gekennzeichnet, dass** die Zellen (29), das zweite flüssige Medium (26) und gegebenenfalls das erste flüssige Medium in dem Reaktionsraum (14) gemischt werden.

**8.** Verfahren nach einem der Ansprüche 5 bis 7, **dadurch gekennzeichnet, dass** vor und nach dem Dosieren in Schritt c) die flüssige Phase (28) 10 Vol.-% bis 80 Vol.-% des inneren Volumens (15) einnimmt.

**9.** Verfahren nach einem der Ansprüche 5 bis 8 **dadurch gekennzeichnet, dass** die Zellen (29) in dem Reaktionsraum (14) in Suspensionskultur vorliegen.

**Claims**

**1.** A device for culturing cells (10), comprising a mixing device (12) and a reaction chamber (14), which is delimited by a reactor wall (16), wherein the reactor wall (16) has at least one connection (18, 20) for the addition of gas and/or liquid and the reaction chamber (14) has an inner volume (15),
wherein the reaction chamber (14) is formed in such a way that the inner volume (15) can be increased by at least 500%, based on a minimum inner initial volume, and
wherein the reactor wall (16) comprises an expandable material, the expandable material having an expandability of at least 200%.

**2.** The device according to claim 1, **characterised in that** the expandable material contains natural rubber, synthetic rubber, polyisobutylene, silicone rubber or mixtures thereof.

**3.** The device according to claims 1 or 2, **characterised in that** the minimum inner initial volume lies in a range of 2 mL to 1 L and can be increased to a maximum inner volume of 10 L to 100 L.

**4.** The device according to one of claims 1 to 3, **characterised in that** the at least one connection (18, 20) for the addition of gas and/or liquid is guided through the expandable material of the reactor wall (16).

**5.** A method for culturing cells in a device according to one of claims 1 to 4, comprising the following steps:

a. optionally, sterilising the reactor wall (16) at a temperature of 80°C to 150°C or by gamma radiation,
b. filling the device, wherein cells (29) and optionally a first liquid medium is filled in the reaction chamber (14),
c. dosing a second liquid medium (26) and/or a gas into the reaction chamber (14), wherein the second liquid medium preferably has a composition which corresponds to that of the first liquid medium (26), and increasing the inner volume (15) of the reaction chamber (14) by at least 500% based on the minimum internal initial volume, wherein the reactor wall (16) has an expandable material and the expandable material in step c. is expanded.

**6.** The method according to claim 5, **characterised in that** the second liquid medium (26) is added continuously and/or incrementally.

**7.** The method according to claims 5 or 6, **characterised in that** the cells (29), the second liquid medium (26) and optionally the first liquid medium are mixed in the reaction chamber (14).

**8.** The method according to one of claims 5 to 7, **characterised in that** before and after the dosing in step c) the liquid phase (28) takes up 10% by volume to 80% by volume of the inner volume (15).

**9.** The method according to one of claims 5 to 8, **characterised in that** the cells (29) are present in the reaction chamber (14) in suspension culture.

# EP 3 630 936 B1

**Revendications**

1. Dispositif de mise en culture de cellules (10) comprenant un dispositif de mélange (12) et une chambre réactionnelle (14) qui est délimitée par une paroi de réacteur (16), la paroi de réacteur (16) présentant au moins un raccord (18, 20) pour l'introduction de gaz et/ou de liquide et la chambre réactionnelle (14) présentant un volume intérieur (15), la chambre réactionnelle (14) étant conçue de sorte que le volume intérieur (15) peut être augmenté d'au moins 500 % rapportés au volume intérieur de départ minimal et la paroi réactionnelle (16) présentant un matériau extensible, qui présente une extensibilité d'au moins 200 %.

2. Dispositif selon la revendication 1, **caractérisé en ce que** le matériau extensible contient du caoutchouc naturel, du caoutchouc synthétique, du polyisobutylène, du caoutchouc de silicone ou leurs mélanges.

3. Dispositif selon les revendications 1 ou 2, **caractérisé en ce que** le volume intérieur de départ minimal est dans une plage entre 2 ml et 11 et peut être augmenté à un volume intérieur maximal de 101 à 1001.

4. Dispositif selon l'une quelconque des revendications de 1 à 3, **caractérisé en ce que** qu'au moins un raccord (18, 20) pour l'introduction de gaz et/ou de liquide traverse le matériau extensible de la paroi réactionnelle (16).

5. Procédé de mise en culture de cellules dans un dispositif conformément à l'une quelconque des revendications de 1 à 4, comprenant les étapes suivantes :

   a. le cas échéant, la stérilisation de la paroi réactionnelle (16) à une température de 80 °C à 150 °C ou par le rayonnement gamma,
   b. remplissage du dispositif, les cellules (29) et, le cas échéant, un premier milieu liquide étant ajoutés dans la chambre réactionnelle (14),
   c. dosage d'un deuxième milieu liquide (26) et/ou d'un gaz dans la chambre réactionnelle (14), le deuxième milieu liquide présentant de préférence une composition correspondante à celle du premier milieu liquide (26), et

   augmentation du volume intérieur (15) de la chambre réactionnelle (14) d'au moins 500 % rapportés au volume intérieur de départ minimal, la paroi réactionnelle (16) présentant un matériau extensible qui est étendu lors de l'étape c.

6. Procédé selon la revendication 5, **caractérisé en ce que** le deuxième milieu liquide (26) est ajouté en continu et/ou progressivement.

7. Procédé selon la revendication 5 ou 6, **caractérisé en ce que** les cellules (29), le deuxième milieu liquide (26) et, le cas échéant, le premier milieu liquide sont mélangés dans la chambre réactionnelle (14).

8. Procédé selon l'une quelconque des revendications de 5 à 7, **caractérisé en ce que** la phase liquide (28) prend de 10 à 80 Vol.-% du volume intérieur (15) lors de l'étape c) avant et après le dosage.

9. Procédé selon l'une quelconque des revendications de 5 à 8, **caractérisé en ce que** les cellules (29) se trouvent en culture de suspension dans la chambre réactionnelle (14).

EP 3 630 936 B1

Fig. 1

Fig. 2

Fig.3

Fig.4

Fig.5

EP 3 630 936 B1

Fig.6

16

**IN DER BESCHREIBUNG AUFGEFÜHRTE DOKUMENTE**

**In der Beschreibung aufgeführte Patentdokumente**

- DE 102013002091
- EP 2543719 A1

**In der Beschreibung aufgeführte Nicht-Patentliteratur**

- Kultivierung von Säugetierzellen. **M. HOWALDT et al.** Bioprozesstechnik. Spektrum Akademischer Verlag, 2011, 410-413
- **T. H. RODRIGUEZ et al.** Considerations for Cell Passaging in Cell Culture Seed Trains. *BMC Proceedings,* 2015, vol. 9 (9), 43
- **B. WRIGHT et al.** A Novel Seed-Train Process. *BioProcess International,* Marz 2015, vol. 13 (3), 16-25